# EUROPÄISCHE PATENTSCHRIFT

(11) **EP 1 156 811 B1**
(45) Veröffentlichungstag und Bekanntmachung des Hinweises auf die Patenterteilung: **28.12.2005**
(21) Anmeldenummer: 00920372.0
(22) Anmeldetag: 01.03.2000
(51) Int. Cl.: A61K 33/30, A61K 33/34, A61K 33/24, G01N 33/68, A61P 25/00

(54) **KUPFERAGONIST, DER AN DIE KUPFERBINDUNGSSTELLE VON AMYLOID VORLÄUFER PROTEIN (APP) BINDET UND/ODER EINE HEMMENDE WIRKUNG AUF DIE FREISETZUNG DES AMYLOID-BETA-PEPTIDS AUSÜBT**
COPPER AGONIST THAT BINDS ON THE COPPER BINDING SITE OF AMYLOID PRECURSOR PROTEIN (APP) AND/OR EXERTS AN INHIBITING EFFECT ON THE RELEASE OF AMYLOID-BETA-PEPTIDE
AGONISTE DU CUIVRE QUI SE LIE AU SITE DE FIXATION DE CUIVRE DE LA PROTEINE PRECURSEUR DE L'AMYLOIDE (APP) ET/OU EXERCE UNE ACTION D'INHIBITION SUR LA LIBERATION DU PEPTIDE BETA-AMYLOIDE

(30) Priorität: 03.03.1999 DE 19909357
(43) Veröffentlichungstag der Anmeldung: 28.11.2001
(73) Patentinhaber: The Genetics Company Inc., 8952 Schlieren (CH)
(72) Erfinder: BEYREUTHER, Konrad, D-69120 Heidelberg (DE); MULTHAUP, Gerd, D-69151 Dilsberg (DE); MASTERS, Colin, L., Clifton Hill, VIC 3068 (AU)
(74) Vertreter: Schüssler, Andrea
(86) Internationale Anmeldenummer: PCT/DE2000/000693
(87) Internationale Veröffentlichungsnummer: WO 2000/051632

(56) Entgegenhaltungen:
- WO-A-00/28331
- WO-A-93/10459
- WO-A-97/18476
- WO-A-98/40071
- WO-A-99/21886
- WO-A-99/45907
- US-A- 5 981 208
- HESSE, LARS ET AL: "The beta-A4 amyloid precursor protein binding to copper." FEBS (FEDERATION OF EUROPEAN BIOCHEMICAL SOCIETIES) LETTERS, (1994) VOL. 349, NO. 1, PP. 109-116., XP000929645
- NOSTRAND: "Zinc(II) selectively enhances the inhibition of coagulation factor XIa by protease Nexin-2/amyloid b protein precursor" THROMBOSIS RESEARCH, Bd. 78, Nr. 1, April 1995 (1995-04), Seite 43-53 XP000933824
- BORCHARDT T. ET AL: "Differential effects of zinc on amyloid precursor protein (APP) processing in copper resistant variants of cultured chinese hamster ovary cells" CELLULAR AND MOLECULAR BIOLOGY, XP000933558
- LI, Q. X. ET AL: "Proteolytic processing of Alzheimers disease bA4 amyloid precursor protein in human platelets" JOURNAL OF BIOLOGICAL CHEMISTRY, Bd. 270, Nr. 23, 9. Juni 1995 (1995-06-09), Seiten 14140-14147, XP000929730
- RUSSO T. ET AL: "Fe65 and the protein network centered around the cytosolic domain of the Alzheimer's b-amyloid precursor protein" FEBS LETTERS, Bd. 434, 1998, Seiten 1-7, XP000929646
- ERMEKOVA K. S. ET AL: "Proteins implicated in Alzheimer disease. The role of Fe65, a new adapter which binds to b-amyloid precursor protein" MOLECULAR AND CELLULAR MECHANISMS OF NEURONAL PLASTICITY, Bd. 10, 1998, Seiten 161-180, XP000933860

## Beschreibung

Die vorliegende Erfindung betrifft ein Verfahren zur Identifizierung von Kupferagonisten, die an die Kupferbindungsstelle des Amyloid-Precursorproteins (APP) binden und/oder eine hemmende Wirkung auf die Freisetzung des Amyloid-Aß-Peptids ausüben, das an der Entstehung der Alzheimer Krankheit beteiligt ist. Die vorliegende Erfindung betrifft ferner die Verwendung von Zn (II) zur Prävention und/oder Behandlung der Alzheimer Krankheit.

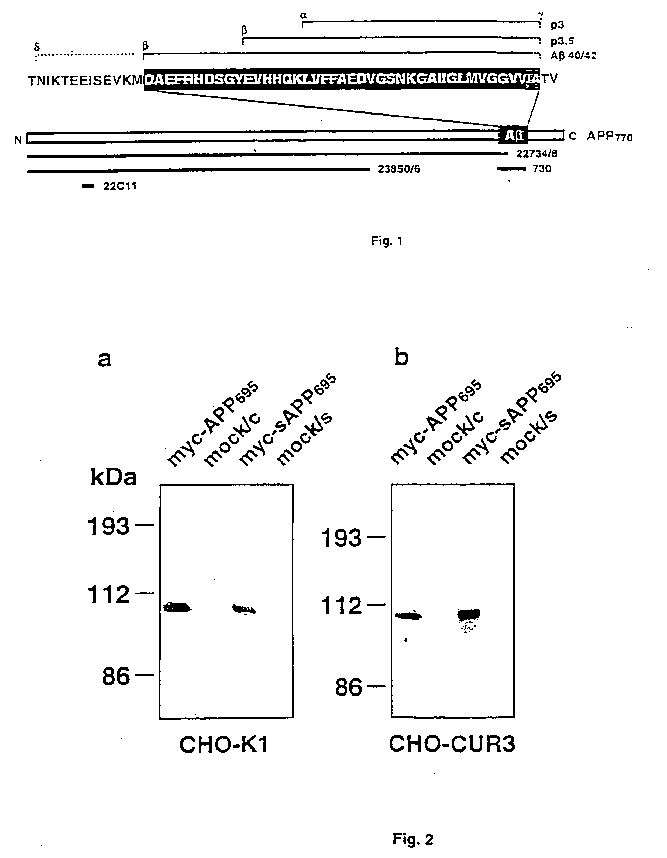

Das β-Amyloidpeptid (βA4, Aβ), der prinzipielle Bestandteil seniler Plaques und das zerebrovaskuläre Amyloid der Alzheimer Krankheit, entstehen durch aufeinanderfolgende proteolytische Spaltungen mit β-Secretase und γ-Secretase aus dem größeren integralen Membran-Amyloid-Precursorprotein (APP), dessen hauptsächliche Isoformen 695 (APP695), 751 (APP751) oder 770 (APP770) Aminosäuren enthalten (Hardy, J., Trends Neurosci 20, S. 154-159 (1997)). Die APP-Prozessierung innerhalb der Aβ-Domäne durch die α-Secretase verhindert die Amyloidbildung und führt zur Freisetzung des p3-Peptids, das die Aβ-Reste 17-40/42 enthält (Figur 1). Die Spaltung von APP mit der α-Secretase oder β-Secretase erzeugt lösliche APP-Fragmente (sAPPα und sAPPβ), die die APP-Ektodomänen darstellen, die in den extrazellulären Raum abgegeben werden.

Die physiologische Rolle von APP ist zwar nicht bekannt, APP teilt jedoch einige Merkmale mit Zelladhäsionsmolekülen und an der Wundheilung beteiligten Molekülen. Dazu zählen beispielsweise die Bindungsstellen von APP für Heparinsulfat, Kollagen, Laminin, Proteasen, Lektine und Metallionen.

Die bisherigen Versuche der Behandlung der Alzheimer Krankheit bestanden darin, die Bildung des Amyloid -Aβ-Peptids durch die Verabreichung von Verbindungen zu blockieren, die die bisher nicht identifizierte β-Secretase oder γ-Secretase blockieren (Hooper et al., Biochem. J. 321, S. 265 - 279 (1997)). Allerdings führt die Hemmung dieser Proteasen zu unerwünschten Nebenwirkungen, da diese Proteasen an der Prozessierung verschiedener Transmembranproteine beteiligt sind. Bisher konzentrierte sich die Behandlung der Alzheimer Krankheit und anderer Demenzformen auf die Verbesserung der Stoffwechselvorgänge im Gehirn und die Substitution bestimmter defizitärer Neurotransmitter oder auf die Hemmung der die Krankheit begleitenden Entzündungsprozesse. Diese Medikamente differenzieren aber nicht zwischen den verschiedenen Demenzformen, sondern zielen auf die bei allen Demenzen zu beobachtenden, sekundären Begleiterscheinungen.

Somit liegt der vorliegenden Erfindung das technische Problem zugrunde, Mittel zu identifizieren, die für eine Prävention oder Behandlung der Alzheimer Krankheit von Nutzen sind.

Die Lösung dieses technischen Problems wurde durch die Bereitstellung der in den Patentansprüchen gekennzeichneten Ausführungsformen erreicht.

Es wurde herausgefunden, daß APP an zwei definierten Stellen spezifisch mit Zn(II) und Cu(II) interagiert. Die Bindung von Cu(II) führt zu einer Oxidation von zwei Cysteinen von APP, was zu der Bildung eines zusätzlichen Cystins führt. Eines der zwei dadurch erzeugten Elektronen reduziert das gebundene Kupfer(II) zu Kupfer(I); das Schicksal des anderen Elektrons ist jedoch bisher nicht geklärt.

Es zeigte sich nun, daß die Zugabe von Kupfer (II) bei Säugerzellen zu einer Steigerung der Freisetzung der APP-Produkte des α-sekretorischen Stoffwechselwegs (sAPPα und p3) und einer Abnahme der Freisetzung der Produkte des β-sekretorischen Stoffwechselwegs (p3.5 und des an der Alzheimer Krankheit beteiligten Aβ) bewirkt. Somit kann die Alzheimer Krankheit wirksam und spezifisch dadurch verhindert oder behandelt werden, daß Verbindungen verabreicht werden, die als Kupferagonisten wirken, d.h., an die Kupferbindungsstelle von APP binden und/oder dessen physiologische Wirkung nachahmen können, d.h., die Bildung des Amyloid-Aβ-Peptids verringern oder verhindern können. Da bei der Kupferbindung durch APP auch sehr toxische Cu(I)-Ionen erzeugt werden, verfügen diese agonistisch wirkenden Moleküle über die zusätzliche Eigenschaft, die Zellen vor einer Schädigung durch die Cu(II)-Bindung von APP zu bewahren und somit die Bildung von reaktiven, durch Kupfer und Wasserstoffperoxid erzeugten Sauerstofformen verhindern zu können.

Somit betrifft eine Ausführungsform der vorliegenden Erfindung die Identifizierung eines Kupferagonisten, der dadurch gekennzeichnet ist, daß er an die Kupferbindungsstelle des Amyloid-Precursorproteins (APP) bindet und/oder die Freisetzung des Amyloid-Aβ-Peptids verringert oder verhindert.

Der hier verwendete Ausdruck "Kupferagonist" betrifft jede Substanz, beispielsweise eine anorganische oder organische Verbindung, die an die Kupferbindungsstelle von APP binden kann und/oder die Freisetzung des Amyloid-Aβ-Peptids verringern oder verhindern kann. Bevorzugte Kupferagonisten sind divalente Metallionen, die in physiologisch hohen Konzentrationen mit Kupfer um die Bindungsstelle am APP-Molekül konkurrieren können, z.B. 20-200 mM Mg(II), bevorzugt 100 mM Mg(II); 20-200 mM Ca(II), bevorzugt 100 mM Ca(II); 0,05-20 mM Zn(II), bevorzugt 100 µM-1 mM Zn(II) etc.. Weitere bevorzugte Kupferagonisten sind Oligopeptide, Oligonukleotide, Oligosaccharide oder Nukleotidanaloga, die hauptsächlich über ihre dreidimensionale Struktur Kupfer nachahmen und gemäß Schlüssel-Schloss-Prinzip an die Kupferbindungsstelle binden. Die Oligopeptide können in einer bevorzugten Ausführungsform auch eine der Kupferbindungsstelle entsprechende Sequenz enthalten und diese dadurch besetzen. Weitere bevorzugte Kupferagonisten werden erhalten aus käuflichen chemischen Substanzbibliotheken oder sind neue chemische Verbindungen (Kombinationschemie) oder niedermolekulare Naturstoffe isoliert aus Mikroorganismen bzw. Pflanzen. Auch diese Verbindungen wirken über ihre dreidimensionale Struktur, die die Eigenschaften und Größe von Kupfer nachahmen ohne dessen negative Eigenschaften zu haben.

Die Wirkung als Kupferagonist beruht also bevorzugt darauf, daß die Substanz spezifisch und mit einer höheren Affinität als Kupferionen an die Cu-Bindungsstelle des APP bindet oder spezifisch an den Sequenzbereich von APP bindet, der für die Kupferbindung verantwortlich ist und dort (sterisch) eine weitere Bindung von Kupfer verhindert bzw. die Kupferionen von dort verdrängt. Kupferagonisten sind solche kompetitiv (für die Kupferbindungsstelle) und nicht-kompetitiv bindenden Liganden, die die physiologische wirkung von Kupfer nachahmen können. Kupferagonisten im Sinne der vorliegenden Erfindung sind auch solche Substanzen, die nicht an die Kupferbindungsstelle binden, aber z.B. die Konformation von APP stabilisieren, die für den Kupfer-APP-Komplex charakteristisch ist, d.h. die physiologische Wirkung von Kupfer an anderen Bindungsstellen (außerhalb der direkten Kupferbindungsstelle) nachahmen können.

Der hier verwendete Ausdruck "Freisetzung des Amyloid-Aß-Peptids verringern oder verhindern" bezieht sich auf eine Wirkung des Kupferagonisten, die als ausreichend bezeichnet werden kann, um eine präventive oder therapeutische Wirkung hinsichtlich der Alzheimer Krankheit zu erzielen.

Der Fachmann ist in der Lage, gemäß üblicher, auf dem Fachgebiet gebräuchlichen Verfahren Stoffe mit der vorstehend beschriebenen Wirkung als Kupferagonisten zu identifizieren und herzustellen. Bei nicht-natürlichen Agonisten (z.B. kleine, organisch-chemische synthetisierte Substanzen, die als sog. Bibliotheken vorliegen), Oligonukleotiden, Oligopeptiden und Nukleotidanaloga erfolgt die Synthese nach den üblichen Verfahren, die dem Fachmann hinreichend bekannt sind. Die Identifizierung der Substanzen als mögliche Agonisten kann über die Affinitätschromatografie erfolgen, z.B. mit der "BIAcore-Technik". Dabei werden vorstehend genannte Verbindungen auf eine Bindung an APP getestet, insbesondere an die Domäne des APP, die die Kupferbindungsstelle beiinhaltet. Anschließend werden die identifizierten Liganden auf einen Effekt auf die APP-Prozessierung im Zellkultursystem getestet, z.B mittels Kompetitionsassays mit geeigneten Antikörpern. Darüber hinaus kann der Fachmann ebenfalls mittels üblicher Verfahren, beispielsweise analog den in den Beispielen 4 und 5 beschriebenen Verfahren testen, ob die identifizierten Agonisten für den Patienten verträglich sind und ggf. eine Dosis-Wirkungskurve aufstellen. Das erfindungsgemäße Verfahren zur Identifikation eines Kupferagonisten, der an die Kupferbindungsstelle von APP bindet und/oder eine hemmende Wirkung auf die Freisetzung des Amyloid-Aß-Peptids ausübt, ist deshalb durch folgende Schritte gekennzeichnet:
(a) Inkontaktbringen von APP oder eines die Kupferbindungsstelle tragenden Fragments davon mit einer sich in Lösung befindenden oder immobilisierten Substanzbibliothek bzw. mit niedermolekularen Substanzen aus Mikroorganismen und/oder Pflanzen,
(b) bei Verwendung einer sich in Lösung befindenden Substanzbibliothek oder flüssigen niedermolekularen Substanzen Immunpräzipitation des Kupfer bindungsstelle/Liganden-Komplexes aus der Lösung mit Antikörpern, die für APP oder das Fragment davon spezifisch sind oder bei Verwendung einer immobilisierten Substanzbibliothek Freisetzung des Liganden aus dem Kupferbindungsstelle/Liganden-Komplex durch Zugabe von Kupfersalzen,
(c) Identifikation des Liganden, und
(d) Selektion von Liganden, die nach Bindung an die Kupferbindungsstelle von APP eine hemmende Wirkung auf die Freisetzung des Amyloid-Aβ-Peptids ausüben,
wobei wahlweise Schritt (d) auch vor Schritt (c) erfolgen kann.

Unter einem "Ligand" ist erfindungsgemäß jede Substanz zu verstehen, die im Rahmen des obigen Verfahrens mit der Kupferbindungsstelle oder anderen Stellen von APP eine Bindung eingeht.

Die oben erwähnten Antikörper sind beispielsweise monoklonale oder polyklonale Antiseren, die APP erkennen und sich für eine Immunpräzipitation eignen. Sie sind käuflich zu erwerben, z.B. Fa. Boehringer Mannheim, Fa. Dianova, Fa. Sigma. Weitere geeignete Antikörper, die sich zum Nachweis von APP, APP-Komplexen und von Aβ40 (Isoform von Aβ mit 40 Aminosäuren; s. Fig. 1) bzw. Aβ42 (Isoform von Aβ mit 42 Aminosäuren, s. Fig. 1) eignen, sind in Ida et al., J. Biol. Chem 271, S. 22908-22914 (1996) sowie Weidemann et al., Cell 57, S. 155-126 (1989) beschrieben.

Als Substanzbibliotheken werden Sammlungen von organischchemischen Verbindungen benutzt, die aufgrund einer Zufallssynthese um "lead"-Substanzen entstanden sind. Diese Bibliotheken können käuflich erworben werden (z.B. Fa. Morphosys, München; Fa. Analytikon, Berlin) oder von chemischen Labors in Kooperation hergestellt werden (z.B. neue chemische Verbindungen mit Kombinationschemie). Niedermolekulare Substanzen aus Mikroorganismen und Pflanzen können mit Hilfe der BIAcore-Technik isoliert werden.

Das Inkontaktbringen geschieht entweder in Lösung oder mit immobilisiertem APP bzw. Fragmenten von APP. Die gebildeten Komplexe werden aus der Lösung mit Hilfe von Antikörpern präzipitiert und anschließend durch Fluoreszenz, Radioaktivität oder analytische Verfahren, wie Massenspektroskopie, Gaschromatografie usw. nachgewiesen.

Ein geeignetes APP-Fragment wird bestimmt, indem man zunächst die Bindung der Testsubstanzen an das gesamte APP testet und anschließend nach und nach kürzere Fragmente testet, die dieselben Bindungseigenschaften besitzen wie das Molekül der vollen Länge.

Eine weitere Möglichkeit der Durchführung des Schrittes b) erfolgt mit Hilfe der Affinitätschromatografie, z.B. BIAcore, d.h. es werden entweder APP-Moleküle oder seine Liganden auf einer Trägeroberfläche immobilisiert. Danach wird das entsprechende Molekül auf diese Oberfläche injiziert und wenn eine Bindung stattgefunden hat, Kupfersalze injiziert, um die Bindung wieder zu lösen. Dieser Vorgang kann mit der Biosensor-Technik direkt verfolgt werden.

In einer bevorzugten Ausführungsform des erfindungsgemäßen Identifikationsverfahrens umfaßt Schritt (d) die Inkubation von stabil mit humanem APP695 transfizierten Säugerzellen mit dem aus den Schritten (a) bis (c) erhaltenen Liganden und die Bestimmung der Produktion des Amyloid-Aβ-Peptids mit polyclonalen oder monoclonalen Antikörpern. Als Assays kommen dabei Immunpräzipitation mit anschließender SDS-Gelelektrophorese, ELISA und Immunpräzipitations-Western-Blot in Frage.

Alternativ kann dabei auch so vorgegangen werden, daß bei Schritt (d) der aus den Schritten (a) bis (c) erhaltene Ligand an transgene Mäuse, die das humane Amyloid-Aβ-Peptid exprimieren, verabreicht wird. Anschließend erfolgt die Gewinnung einer Probe aus dem ZNS oder dem Blut aus der Schwanzvene und die Bestimmung der Produktion des Amyloid-Aβ-Peptids mit polyclonalen oder monoclonalen Antikörpern (z.B. Fa. Dianova, Fa. Boehringer Mannheim, Fa. Sigma und eigene Antikörper [Ida et al., J. Biol. Chem, 271, S. 22908-22914, 1996]). Dieses alternative Verfahren stellt einen in-vivo Test der identifizierten Liganden dar. Dabei ist die Vorgehensweise (einschl. der benutzen Methoden) sehr ähnlich zu dem vorstehend beschriebenen Zellkulturexperiment. Anstatt die gebildeten APP-Fragmente im Zellkulturüberstand oder intrazellulär nachzuweisen, erfolgt der Nachweis dieser Fragmente bei Mäusen im Serum. Die Nachweisverfahren entsprechen denen des Zellkulturexperiments.

Die vorliegende Erfindung ferner die Verwendung von Zn(II) zur Herstellung eines Arzneimittels zur Prävention oder Behandlung der Alzheimer Krankheit.

Die Erfindung wird weiter anhand der Figuren beschrieben, welche zeigen:

### Fig.1: Prozessierung des Amyloid-Precursorproteins (APP770) zu Aβ, p3.5 und p3.

Einbuchstabencode; die weißen Buchstaben bezeichnen die Aminosäuresequenz von Aβ1-40/42. Die ungefähre Lage der Epitope für die Antikörper ist unterhalb der Sequenz mit schwarzen Balken angegeben.

### Fig.2: Immunpräzipitation von c-myc-getaggtem APP695

Aus dem Lysat (myc-APP695) und dem konditionierten Medium (myc-sAPP695) des Wildtyps (a, CHO-K1) und aus Kupferresistenten CHO-Zellen (b, CHO-CUR3) wurde mit polyclonalen c-myc-Antikörpern 18/47 eine Immunpräzipitation vorgenommen. Die präzipitierten Proben wurden nach Transfer auf eine Nitrozellulosemembran mit dem monoclonalen APP-Antikörper 22C11 immungeblottet. Kontrolle: Medium ("mock/s") und Lysat ("mock/c) von "mock"-transfizierten Zellen .

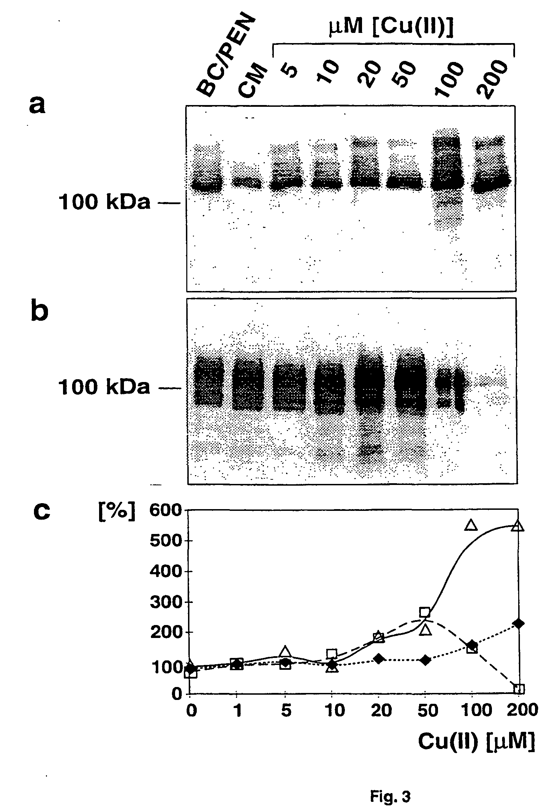

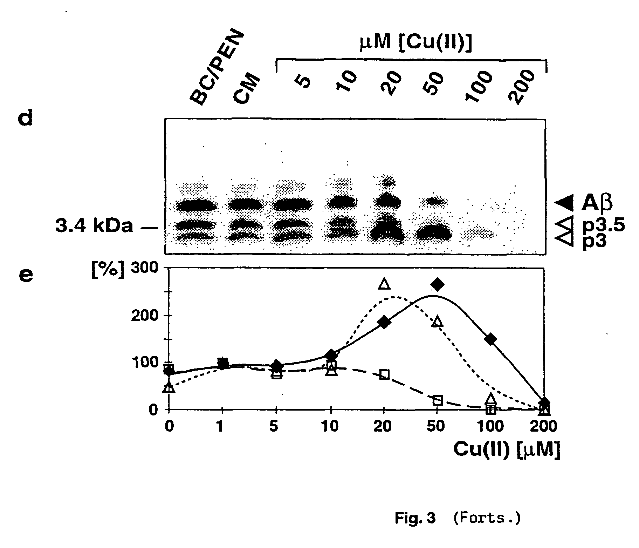

### Fig. 3: Immunpräzipitation von Holo-APP695 aus dem Lysat (a), löslichem APP695 (b) und Aβ und p3 aus dem Medium (d) von metabolisch markierten CUR3-Zellen mit anti-APP (22734/6 ; polyklonales Serum aus Kaninchen, das durch Immunisierung mit in Bakterien produziertem APP gewonnen wurde, s. Fig. 1) oder anti-Aβ (730; polyklonales Serum aus Kaninchen, das durch Immunisierung mit synthetisch hergestelltem Aβ40-Peptid gewonnen wurde) nach vierstündiger Inkubation in der Gegenwart der Kupferchelatbildner Bathocuproin (BC) und D-Penicillamin (PEN) in normalem Medium (CM; Basalmedium-Kupfer enthaltend) oder in Medium, das die angegebenen Kupferkonzentrationen enthielt

Das Auftreten von sAPP695 als Dublette (b) bei höheren Kupferkonzentrationen beruht auf seinem unterschiedlichen Sialinsäure-Gehalt. Ein etwas über der Aβ-Bande laufendes Fragment (d) tritt bei geringen Kupferkonzentrationen auf und wird vermutlich durch Spaltung mit δ- und γ-Secretase erzeugt (siehe auch Figur 1). Die in diesem Experiment gemessenen relativen Mengen an cAPP (offene Dreiecke), sAPP (offene Quadrate) und gesamtem sezernierten Protein (schwarze Karos) wurden als Prozentsatz an Radioaktivität quantifiziert, der bei Basalmedium-Kupferkonzentrat in c) erhalten wurde; Aβ (offene Quadrate), p3 (offene Dreiecke) und sAPP (schwarze Karos; die von demselben Experiment wie in (c) stammenden Daten sind zum besseren Vergleich gezeigt) in (e). Die Daten entsprechen den Durchschnittswerten aus drei unabhängigen Experimenten. "0" µM und "1" µM Kupfer in (e) bezeichnen Inkubationsbedingungen für Kupferentzug (PEN/BC) und Kupfer im Basalmedium (CM; 0,8 µM) .

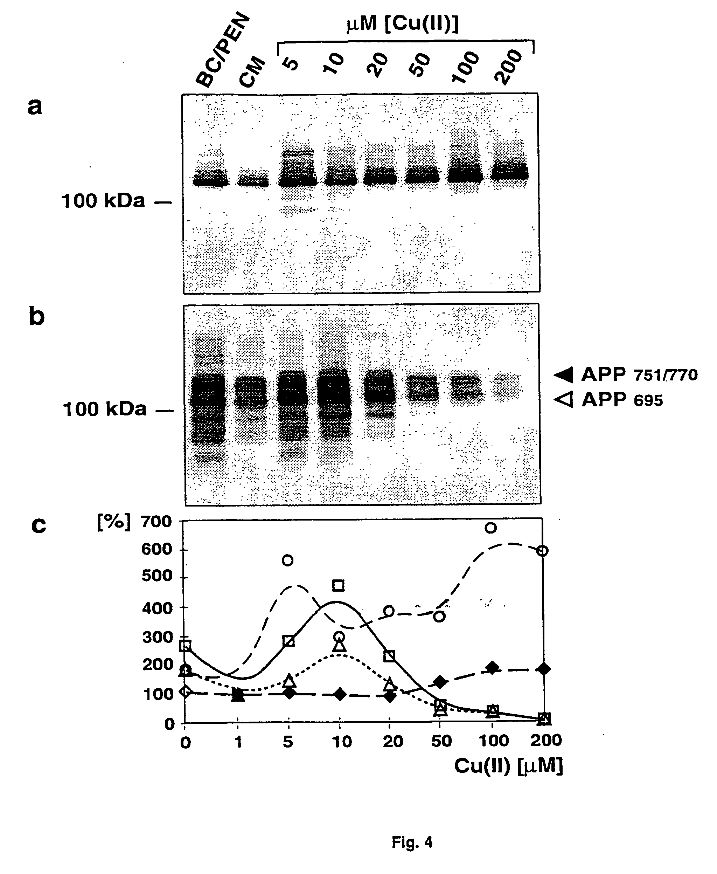

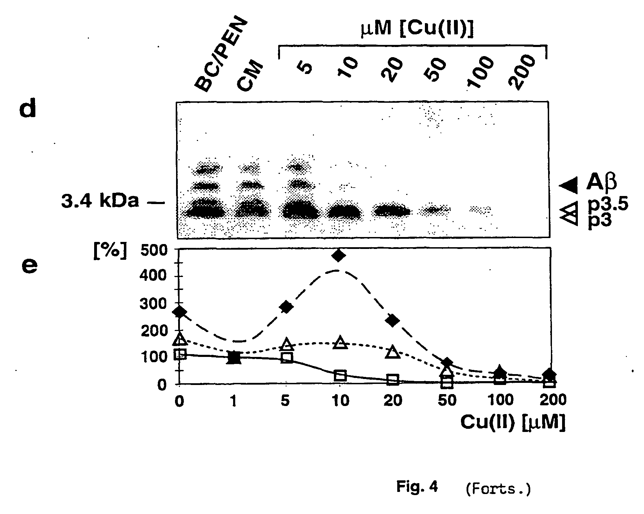

### Fig. 4: Immunpräzipitation von Holo-APP695 aus dem Lysat (a), löslichem APP695 (b) und Aβ und p3 aus dem Medium (d) von metabolisch markierten K1-Zellen mit anti-APP (22734/6) oder anti-Aβ (730) nach vierstündiger Inkubation in der Gegenwart der Kupferchelatbildner Bathocuproin (BC) und D-Penicillamin (PEN) in normalem Medium (CM; Basalmedium-Kupfer enthaltend) oder in Medium, das die angegebenen Kupferkonzentrationen enthielt, quantifiziert wie in Figur 3.

Der endogene Spiegel von sAPP751/770 ist in K1-Zellen höher als in CUR3-Zellen (siehe Figur 2) und wird auch durch die Kupferkonzentration beeinflußt und wurde daher quantifiziert. Die Daten stellen die Durchschnittswerte aus zwei unabhängigen Experimenten dar. Die in diesem Experiment gemessenen relativen Mengen an cAPP (offene Kreise), sAPP695 (offene Quadrate), sAPP751/770 (offene Dreiecke) und an gesamtem sezerniertem Protein (schwarze Karos) sind in c) quantifiziert. Aβ (offene Quadrate), p3 (offene Dreiecke) und sAPP695 (schwarze Karos; die Daten des gleichen wie in c) gezeigten Experiments in (e). "0" µM und "1" µM Kupfer in (e) bezeichnen Inkubationsbedingungen für Kupferentzug (PEN/BC) und Kupfer im Basalmedium (CM; 0,8 µM) . Ein etwas über der Aβ-Bande laufendes Fragment (d) tritt bei geringen Kupferkonzentrationen auf und wird vermutlich durch Spaltung mit δ- und γ-Secretase erzeugt (siehe auch Figur 1).

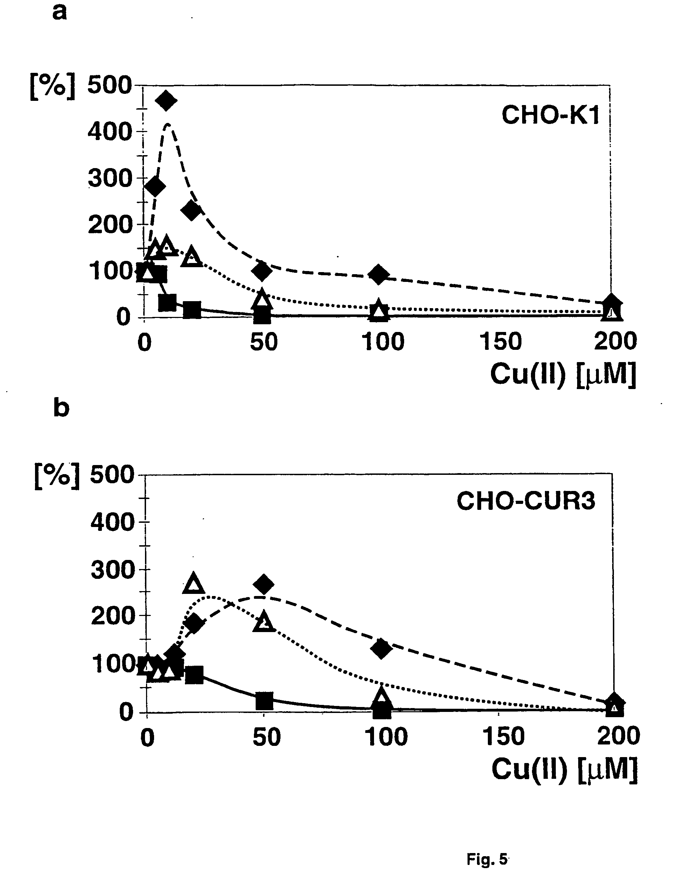

### Fig. 5: Quantifizierung der relativen Spiegel an sAPP695 p3 und Aß die von konditioniertem Medium von CHO-K1-Zellen (a) und CHO-CUR3-Zellen (b) immunpräzipitiert wurden

Die Zunahme an sAPP695 (schwarze Karos) erreichte in K1-Zellen ein Maximum bei 10 µM Cu(II) (a) und in CUR3-Zellen bei 50 µM Cu(II) (b); danach erfolgte wieder eine Abnahme. Die Veränderung des sAPP-Spiegels (offene Dreiecke) trat im Vergleich zu den Veränderungen mit dem p3-Protein, das den Maximalspiegel bei etwas geringeren Cu(II)dosierungen erreichte, später ein, was höchstwahrscheinlich durch die höhere Halbwertszeit im Vergleich zu sAPP bedingt ist. Im Gegensatz dazu stiegen die Aβ-Spiegel (schwarze Quadrate) in beiden Zellinien bei Cu(II)-Konzentrationen unter dem Basalspiegel drastisch an. Der erste Datenpunkt wurde bei der basalen Kupferkonzentration ermittelt (0,8 µM Cu(II)).

Die nachstehenden Beispiele veranschaulichen die Erfindung.

### Beispiel 1: Allgemeine Verfahren

### Zellinien und Transfektionen

CHO-Zellen wurden mit einem c-myc-getaggtem APP695-Vektor (Peraus et al., J. Neurosci 17, S. 7714-7724 (1997)) oder mit dem Expressionsvektor (pcDNA3) mittels einer hocheffizienten Kalziumphosphat-Transfektion (Chen et al., Biotechniques 6, S. 632-638 (1988) transfiziert. Die pSP65 (Fa. Invitrogen)-N-tag APP 695-DNA wurde in den Vektor pcDNA3 (Invitrogen/ITC Biotechnology, Heidelberg) unter Verwendung der SmaI- bzw. EcoRV-Clonierungsstellen cloniert. Die Transfektionseffizienz wurde mittels Immunpräzipitation von APP mit polyclonalem Kaninchen-anti-c-myc-Antikörper (18/47; erzeugt gegen die Aminosäuresequenz EQKLISEEDL der c-myc-Sequenz; von Eurogentec, Seraing, Belgien) und Immunblotts mittels dem monoclonalen Maus-Antikörper 22C11 (Boehringer Mannheim) in dem "ECL"-Nachweissystem (Amersham, Braunschweig) überprüft. Die parentalen CHO-K1-Zellen sowie die Kupfer-resistente Zellvariante CUR3 wurden bei 37°C in "Eagles"-Medium (BME) gezüchtet, dem 2 mM L-Glutamin, 0,1 mM Prolin, 20 mM HEPES und 10% fötales Kälberserum (Boehringer Ingelheim) zugesetzt waren. Die Grundkonzentration an Kupfer betrug 0,8 *µ*M. CUR3-Zellen wurden in einem Medium mit 200 *µ*M zugegebenem Kupfer gezüchtet.

### Metabolische Markierung und Immunpräzipitation

Stabil transfizierte CHO-Zellen einer Kulturschale (60 mm) wurden mit 2 ml essentiellem Minimalmedium (MEM), das kein Methionin enthielt (Sigma, München) und mit 220 *µ*Ci [³⁵S] Methionin (Amersham, Braunschweig) und 5% N₂ supplementiert worden war, 4 Stunden behandelt. Das konditionierte Medium (CM) und die Zellen wurden geerntet und immunpräzipitiert. Zuvor wurden die Proteinkonzentrationen mittels eines "BioRad"-Proteinassays (Bradford et al., Anal. Biochem. 72, S. 248-254 (1976)) bestimmt oder die radioaktiv markierten Proteine wurden mit 10% Trichloressigsäure (TCA) präzipitiert und das eingebaute [³⁵S]Methionin gemessen (Beckman LS 6000IC). Die Zellen wurden in Extraktionspuffer lysiert, der 50 mM Tris-HCl (pH-Wert 7,5), 150 mM NaCl, 2 mM EDTA, 2% Triton X-100, 2% NP40, 10 *µ*g/ml Aprotinin und 10 µg/ml Leupeptin enthielt. Zur Entfernung von Zelltrümmern wurden das Lysat und das Medium 10 Minuten bei 13.000 x g zentrifugiert und das Medium auf 25 mM Tris-HCl (pH-Wert 8,5), 1 mM PMSF, 10 µg/ml Aprotinin, 10 µg/ml Leupeptin, 0,5% Triton X-100 und 0,5% NP40 eingestellt. Die solubilisierten Proteine wurden in 100 mM Tris-HCl (pH-Wert 7,5), 300 mM NaCl und 4 mM EDTA 1:2 verdünnt. Die Überstände wurden über Nacht bei 4°C mit APP-Antikörpern bei "end-over-end"-Rotation in in einem Überkopfschüttler inkubiert. In einigen Experimenten wurden die erhaltenen Überstände im Anschluß daran mit Aβ-Antiserum analysiert. Immunkomplexe wurden mit Protein-A-Sepharose gewonnen und wie in (Weidemann et al., Cell 57, S. 115-126 (1989)) beschrieben analysiert.

### Antikörper und Elektrophorese

Zum Nachweis von APP gegen rekombinantes Fd-APP770 gerichtete polyclonale Kaninchen-Antikörper (Antiserum 22734/6 gegen die Aminosäurereste 18-687 von APP770 oder Antiserum 23850/6 gegen die AAP-Aminosäurereste 18-491 (Figur 1) wurden zur Präzipitation 1:500 verdünnt. Die Antiseren wurden nach den dem Fachmann bekannten Standardmethoden im Labor hergestellt. Aβ, p3.5 und p3 wurden von dem polyclonalen Antikörper 730 (Verdünnung 1:50; Aβ, p3.5 und p3 wurden gleichermaßen präzipitiert) erkannt, der gegen ein synthetisches Peptid erzeugt worden war, das den Aβ-Aminosäureresten 1-40 entsprach (Figur 1). Aβ und seine Derivate wurden durch 10-20% Tris-Tricin-Polyacrylamidgele und APP durch 7% Tris-Glycin- bzw. 7% Tris-Tricin-Polyacrylamidgele aufgetrennt. Für Immunblotts wurden Zellextrakte und extrazelluläres Medium mit 10% TCA präzipitiert, mit Azeton gewaschen und in Probenpuffer gelöst. Nach der Elektrophorese wurden die Gele wie in (Simons et al., J. Neurosci **16**, S. 899-908 (1996)) beschrieben weiterverarbeitet und mit dem "Fuji-Bas-PhosphorImager"-System quantitativ ausgewertet. Die Daten wurden als Mittelwerte +/-Standartabweichung ausgedrückt und, falls nicht anders angegeben, repräsentieren diese das Ergebnis von mindestens zwei getrennten Versuchen.

### Wirkstoffbehandungen und LDH-Assay

Während des Markierungszeitraums wurden zu dem Zuchtmedium Kupfer- oder Zinkchlorid (Konzentrationen: 5 µM bis 200 µM) gegeben. D (-) - Penicillamin (PEN; Sigma), Bathocuproindisulfonat (BC; Aldrich) und 1,10-Phenanthrolin (PEN; Sigma) wurden zu dem Medium bis zu einer Endkonzentration von 100 *µ*M (PEN und BC) bzw. 200 *µ*M (PEN) gegeben. Die Lebensfähigkeit der Zellen wurde anhand des Laktatdehydrogenase(LDH)-Austritts in die Kultur gemessen (41). Der LDH-Austritt wurde als Enzymaktivität (Einheiten/Liter) nach 2 und 4 Stunden in dem Medium von 70% konfluenten Monolayern von CHO-Zellen in nach Dulbecco modifiziertem "Eagle"-Medium (DMEM) gemessen.

### Beispiel 2: APP-Expression in Zellen, die mit APP695 codierender cDNA transfiziert waren

Zur Untersuchung der Rolle von Kupfer auf die APP-Prozessierung bei abnehmenden Kupferkonzentrationen bzw. ansteigenden Kupferkonzentrationen (0 - 200 *µ*M) wurden die parentale CHO-K1-Zellinie und die Kupfer-resistente CUR3-Zellinie verwendet. CUR3-Zellen wurden zur Untersuchung der Rolle von intrazellulärem Kupfer auf die APP-Prozessierung verwendet. Im Vergleich zu CHO-K1-Zellen zeigen CUR3-Zellen geringere intrazelluläre Kupferkonzentrationen, was durch ein verstärktes Ausströmen von Kupfer als Folge einer um den Faktor 70 erhöhten Konzentration der "Menkes P-type ATPase copper efflux pump " (intrazelluläre Kupferpumpe/intrazelluläres Kupferexport-System) hervorgerufen wird.

Nach Transfektion von CHO-K1- und CUR3-Zellen mit cDNA, die APP695 mit einem N-terminalen c-myc-Epitop codierte (Figur 1), war APP-Expression mittels Immunpräzipitation mit polyclonalem c-myc-Antiserum und darauffolgenden Immunblotts mit dem monoclonalen Antikörper 22C11 leicht nachweisbar (Figur 2a,b). Der polyclonale c-myc-Antikörper immunpräzipitierte c-mycgetaggtes APP695 mit einem relativen MG von 105 kDa aus dem Zellysat (cAPP695) und dem konditionierten Medium (sAPP695). Entsprechend den Erwartungen konnte c-myc-APP695 weder in dem Lysat noch in dem Medium von Zellen, die nur mit pcDNA3 transfiziert worden waren, nachgewiesen werden.

### Beispiel 3: Abnahme der Aß-Konzentration in Abhängigkeit von ansteigenden Kupferkonzentrationen

Zur Untersuchung der Regulation des APP-Metabolismus durch Kupferionen wurden mit APP695 stabil transfizierte CHO-Zellen 4 Stunden mit ³⁵S-markiertem Methionin in Gegenwart von verschiedenen Kupferkonzentrationen markiert und nach Immunpräzipitation mit polyklonalen Antikörpern, die APP erkennen (22734/6) bzw. die Aβ und p3 erkennen (730) wurde die Sezernierung der APP-Ektodomäne, von Aβ und p3 gemessen. Zellysate von CUR3-Zellen zeigten eine Bande bei 105 kDa, die dem unreifen cAPP695-Holoprotein entspricht (Figur 3a). In konditioniertes Medium abgegebene APP695-Derivate wurden als Banden von 105 und 97,5kDa (Figur 3b) nachgewiesen, wobei sich diese wahrscheinlich dadurch unterscheiden, daß die obere Bande einen höheren Gehalt an Sialinsäuren aufweist. Während des ganzen Experiments zeigten die Zellinien keine verringerte Lebensfähigkeit.

Bei der Inkubation von CUR3-Zellen mit ansteigenden Kupferkonzentrationen stieg die Konzentration des APP-Holoproteins und von löslichem APP signifikant an. Im Vergleich zu dem Basalmedium wurde ein Maximum an sAPP mit 265% bei 50 µM Cu(II) erreicht, bevor eine allgemeine Produktion von sezernierten Proteinen induziert wurde (Figur 3c; Tabelle 1). Die Sezernierung von p3, dem C-terminalen Gegenstück zu sekretorischem APP, das durch Spaltung mit α-Secretase erzeugt wird, stieg zwischen 20 *µ*M und 50 *µ*M im Medium auf 270% an (Figur 3d; Tabelle 1). Die Immunpräzipitation der von dem Aß-Bereich von APP stammenden radioaktiv markierten Peptide erfolgte mit dem Kaninchen-Antiserum 730, das Aß (4,5 kDa), p3.5 (3, 5 kDa) und p3 (3 kDa) erkennt (Figur 1). Ein Überschreiten einer Kupferkonzentration von. 50 µM im Medium beeinflußte den allgemeinen Proteimmetabolismus und der Prozentsatz an cAPP war deutlich höher bei 550% (Figur 3c; Tabelle 1). Im Gegensatz dazu war die Aß-Konzentration stark erniedrigt, bei 50 µM Kupfer waren lediglich 20% der Basalkonzentration vorhanden und über 100 µM war in CUR3-Zellen Aβ kaum nachweisbar (Figur 3d,e). Dies wurde auch für p3.5 ermittelt (Figur 3d), was die frühere Beobachtung bestätigt, daß p3.5 das Produkt eines alternativen β-Secretase-Stoffwechsels ist.

**Tabelle 1:**

| Modulation der APP-Prozessierung in CHO-Zellen* | | | | |
|---|---|---|---|---|
| | sAPP695c | APP695 | p3 | Aβ |
| CUR3 (50 µM Cu (II)) | 265 | 550 | 270 | 20 |
| CUR3 (Cu(I)/Cu(II)Entzug) | 77 | 205 | 50 | 100 |
| K1 (10 µM Cu (II)) | 475 | 295 | 154 | 20 |
| K1 (Cu(I)/Cu(II)Entzug) | 170 | 190 | 171 | 100 |

| | | | | |
|---|---|---|---|---|
| *[%] Zunahme/Abnahme im Vergleich zu Standardbedingungen | | | | |

### Beispiel 4: Stimulation der Aβ-Peptidproduktion durch Komplexbildung von Kupfer(I)-Ionen mit Bathocuproin bzw. der Kupfer(II)-Ionen durch Penicillamin

Die folgenden Untersuchungen wurden durchgeführt, um zu überprüfen, ob cAPP, sAPP und nachfolgende Abbauprodukte durch einen Kupfermangel moduliert werden. Um zu untersuchen, ob Kupfer(II) für die APP-Expression und die Prozessierung erforderlich ist, wurde Kupfer(II) mittels des Kupfer(II)-Chelatbildners D-Penicillamin (PEN) und Kupfer(I) mittels des nicht Zell-permeablen Kupfer(I)-Chelatbildners Bathocuproindisulfonsäure entfernt. Es zeigte sich, daß die Produktion an Aβ und p3.5 nicht beeinflußt wurde, während allerdings die Konzentration von sAPP und p3 auf 77% bzw. 50% erniedrigt war (Figur 3d,e; Tabelle 1). Dies zeigt, daß die Kupfer(II)-induzierten Veränderungen auf einer spezifischen Modulation des APP-Metabolismus durch Kupfer beruhen.

### Beispiel 5: Untersuchung der endogen exprimierten KPI enthaltenden APP751/770-Isoformen in CHO-K1-Zellen.

Die endogen exprimierten KPI (Protease-Inhibitordomäne vom Kunitz-Typ, die durch alternatives Spleißen in APP751 und APP770 gebracht wird) enthaltenden Isoformen APP751/770 wurden in CHO-K1-Zellen durch Immunpräzipitation von mit [³⁵S] Methionin markierten Proteinen aus dem Zellysat und konditionierten Medien mit polyclonalem APP-Antiserum 22734 (Figur 1; Figur 4a,b) untersucht. Die Sezernierung von KPI-AAP entsprach der von c-myc-APP695 (Figur 4). Zwei Hauptbanden mit einem relativen Molekulargewicht von 130 kDa und 105 kDa entsprechen sAPP751/770 bzw. sAPP695. Letzteres konnte auch mit anti-c-myc-Antiserum präzipitiert werden. Bei Inkubation der CHO-K1-Zellen mit ansteigenden Kupferkonzentrationen stiegen die APP-Spiegel und p3-Spiegel signifikant (APP: cAPP auf 295%, sAPP695 auf 475% und sAPP751/770 auf 275% bei 10 *µ*M Kupfer und p3 auf 154% bei 10 *µ*M Kupfer; Figur 4a-e; Tabelle 1). Gleichzeitig verringerte sich die Aß-Produktion dramatisch bis unter die Nachweisgrenze (Figur 4d,e). In Gegenwart von Kupferchelatbildnern betrugen die sAPP695-Sezernierung 170% (Figur 4b,c), die sAPP751/770-Sezernierung 190% (Figur 4b,c; Tabelle 1) und die p3-Sezernierung 171% (Figur 4d,e; Tabelle 1).

Weiter wurde der relative Spiegel an sAPP695, p3 und Aβ, die von konditioniertem Medium von CHO-K1-Zellen (a) und CHO-CUR3-Zellen (b) immunpräzipitiert wurden, quantifiziert. Das Ergebnis ist in Fig. 5 gezeigt. Die Zunahme an sAPP695 (schwarze Karos) erreichte in K1-Zellen ein Maximum bei 10 µM Cu(II) (a) und in CUR3-Zellen bei 50 µM Cu(II) (b); danach erfolgte wieder eine Abnahme. Die Veränderung des sAPP-Spiegels (offene Dreiecke) trat im Vergleich zu den Veränderungen mit dem p3-Protein, das den Maximalspiegel bei etwas geringeren Cu(II)dosierungen erreichte, später ein, was höchstwahrscheinlich durch die höhere Halbwertszeit im Vergleich zu sAPP bedingt ist. Im Gegensatz dazu stiegen die Aß-Spiegel (schwarze Quadrate) in beiden Zellinien bei Cu(II)-Konzentrationen unter dem Basalspiegel drastisch an. Der erste Datenpunkt wurde bei der basalen Kupferkonzentration ermittelt (0, 8 µM Cu(II)) .

### Beispiel 6: Identifizierung von Kupferagonisten für APP mit Inhibitorwirkung auf die Freisetzung des Anyloid-Aβ-Peptids

Das Kupfer-bindende Peptid von APP und APLP2 (homologes Protein zum APP, das ebenso wie APP und APLP1 zur APP-Genfamilie gehört), APPN262 (eine künstlich erzeugte, C-terminal verkürzte Form von APP, das aus den N-terminalen 262 Resten des APP besteht) und weitere künstlich erzeugte APP-Formen, die sukzessive um einzelne Domänen vom C-Terminus verkürzt sind), die Kupferbindungsstelle tragenden Varianten von APP und APLP2 sowie Fragmente davon wurden mit einer unterschiedlichen Konzentration an Zn(II) in Kontakt gebracht. Die verwendeten Zn²⁺-Konzentrationen betrugen 10 *µ*m, 50 *µ*M, 100 *µ*M und 200 *µ*M. Im Anschluß daran wurde untersucht, ab welcher Konzentration an Zn(II) die Freisetzung des Aβ-Peptids gehemmt werden kann. Dazu wurden folgende Versuche durchgeführt:
(1) CHO-Zellen, die stabil mit humanem APP695 transfiziert waren, wurden in MEM Medium mit den oben genannten Konzentrationen an Zn(II) (10 bis 200 *µ*M in PBS) inkubiert. Als Kontrollen wurden auch CHO-Zellen mit Kupferionen (10 bis 50 µM in PBS) oder ohne jeden Zusatz inkubiert. Die Produktion von Aβ(Gesamt), Aβ40 und Aβ42 wurde qualitativ und quantitativ mit polyclonalen und monoclonalen Antikörpern bestimmt. Der Nachweis von APP, Aβ und p3 erfolgte nach biosynthetischer Markierung mit ³⁵S-Methionin wie folgt. Die stabil transfizierten CHO-Zellen, die wie oben erwähnt mit den jeweiligen Substanzen inkubiert wurden, wurden mit 220 µCi ³⁵S-Methionin für 4 Std. inkubiert. Konditioniertes Medium und Zellen wurden geerntet und die gelösten Proteine für die Immunpräzipitation eingesetzt. Zuvor wurden die Proteinkonzentrationen mit dem Protein-Nachweis-Kit der Fa. BioRad bestimmt und die Menge an eingebauter Radioaktivität über Szintilationszählung ermittelt. Die Zellen wurde in Extraktionspuffer (50 mM Tris-HCl, pH 7,5, 150 mM NaCl, 2 mM EDTA, 2% Triton X-100, 2 % NP40, 1 mM PMSF, 10 µg/ml Aprotinin und 10 µg Leupeptin) lysiert. Zelltrümmer wurden für 10 Min. bei 13.000 x g abzentrifugiert und der Überstand auf 25 mM Tris-HCl, pH 8,5, 1 mM PMSF, 10 µg/ml Aprotinin, 10 µg/ml Leupeptin, 0,5% Triton X-100 und 0,5% NP40 eingestellt. Die Proteinlösung wurde 1:2 verdünnt mit 100 mM Tris-HCl, pH 7,5, 300 mM NaCl und 4 mM EDTA. Die Überstände wurden mit den oben genannten polyklonalen APP- und Aß-Antikörpern über Nacht bei 4°C inkubiert, die gebildeten Immunkomplexe mit Protein A Sepharose isoliert und analysiert gemäß dem in Ida et al., J. Biol. Chem. 271, S. 22908-22914 beschriebenen Verfahren.
   Es zeigte sich, daß mit einer Konzentration ab 50 µM Zn (II) die Aβ-Produktion dramatisch reduziert werden konnte. Eine erniedrigte Sezernierung von APP in CHO-K1-Zellen und in CUR3-Zellen zeigte sich bei den Zn(II)-Konzentrationen über 50 µM, mit einer um 60% verminderten Aβ-Produktion bei 50 µM Zn(II) für CUR3-Zellen (K1: 20%), 90% bei 100µM Zn(II) (K1:305) und 99% bei 200µM Zn(II) (K1: 60%). Über 200 µM Ca(II) war keine weitere Abnahme der Aß-Produktion mehr zu verzeichnen, wobei hier die Nachweisgrenze erreicht wurde.
(2) Aus transgenen Mäusen, die das Aβ-Peptid mit der humanen Sequenz exprimierten, wurden primäre Neuronen isoliert und deren Aβ-Produktion wie unter (1) beschrieben bestimmt.
(3) Transgenen Mäuse (siehe (2)) wurden die oben genannten Zn(II)-Konzentrationen in PBS oral, i.v., i.p., s.c. und i.c. appliziert und die Aβ-Produktion wurde im ZNS und im Blut (das aus der Schwanzvene entnommen wurde) analog wie vorstehend unter (1) beschrieben bestimmt. Das aus der Schwanzvene der Mäuse gewonnene Serum (150-200 µl) wird mit PBS-Puffer auf 500 µl Volumen aufgefüllt und mit 5 µg monoklonalem Antikörper WO-2 (Ida et al., s.o.) und 20 µl einer 1:1 Suspension von Protein G-Sepharose über Nacht bei 4°C auf einem Überkopfschüttler inkubiert. Der resultierende Überstand wird anschließend mit polyklonalen Antikörpern (22734/6) gegen APP inkubiert und die Immunkomplexe wie vorstehend unter (1) beschrieben analysiert. Die Aβ-Immunkomplexe werden mit Hilfe 12-%iger Bis-Tricin Novex Gele entsprechend den Angaben des Herstellers aufgetrennt und der relevante Molekulargewichtsbereich nach der Übertragung der Proteine auf ein Nitrozellulosefilter (380 mA bie 4°C für 40 Minuten) mit dem monoklonalen Antikörper WO-2 mit der ECL-Technik gemäß Ida et al.,s.o. analysiert. Auch hier war das Ergebnis, daß ab einer Konzentration von 50 µM Zn(II) eine Abnahme der Aβ-Produktion erzielt werden konnte.

## Patentansprüche

1. Verfahren zur Identifikation eines Kupferagonisten, der an die Kupferbindungsstelle von APP bindet und eine hemmende Wirkung auf die Freisetzung des Amyloid-Aß-Peptids ausübt, das durch folgende Schritte **gekennzeichnet** ist:
(a) Inkontaktbringen von APP oder eines die Kupferbindungsstelle tragenden Fragments davon mit einer sich in Lösung befindenden oder immobilisierten Substanzbibliothek bzw. mit niedermolekularen Substanzen aus Mikroorganismen und/oder Pflanzen,
(b) bei Verwendung einer sich in Lösung befindenden Substanzbibliothek oder flüssigen niedermolekularen Substanzen Immunpräzipitation des kompetitiven oder nicht-kompetitiven Kupferbindungsstelle/Liganden-Komplexes aus der Lösung mit Antikörpern, die für APP oder das Fragment davon spezifisch sind oder bei Verwendung einer immobilisierten Substanzbibliothek Freisetzung des Liganden aus dem Kupferbindungsstelle/Liganden-Komplex durch Zugabe von Kupfersalzen,
(c) Identifikation des Liganden, und
(d) Selektion von Liganden, die nach Bindung an die Kupferbindungsstelle von APP eine hemmende Wirkung auf die Freisetzung des Amyloid-Aβ-Peptids ausüben,
wobei wahlweise Schritt (d) auch vor Schritt (c) erfolgen kann.

2. Verfahren nach Anspruch 1, wobei Schritt (d) die Inkubation von stabil mit humanem APP695 transfizierten Säugerzellen mit dem aus den Schritten (a) bis (c) erhaltenen Liganden und die Bestimmung der Produktion des Amyloid-Aβ-Peptids mit polyclonalen oder monoclonalen Antikörpern umfaßt.

3. Verfahren nach Anspruch 1, wobei Schritt (d) die Verabreichung des aus den Schritten (a) bis (c) erhaltenen Liganden an transgene Säuger, die das humane Amyloid-Aβ-Peptid exprimieren, umfaßt, die Gewinnung einer Probe aus dem ZNS oder dem Blut und die Bestimmung der Produktion des Amyloid-Aβ-Peptids mit polyclonalen oder monoclonalen Antikörpern.

4. Verwendung von Zn (II) zur Herstellung eines Arzneimittels zur Prävention oder Behandlung der Alzheimer Krankheit.

## Claims

1. A method of identifying a compound as a copper agonist which binds to the copper binding site of APP and exerts an inhibitory effect on the release of the amyloid Aβ peptide, which is **characterized by** the following steps of:
(a) contacting of APP or a fragment thereof canying the copper binding site with a dissolved or immobilized substance library or low molecular natural substances from microorganisms and/or plants,
(b) when a dissolved substance library or liquid low molecular substances are used, immunoprecipitation of the competitive or non-competitive copper binding site/ligand complex from the solution with antibodies specific to APP or the fragment thereof or, when an immobilized substance library is used, release of the ligand from the copper binding site/ligand complex by the addition of copper salts,
(c) identification of the ligand, and
(d) selection of ligands which after binding to the copper binding site of APP exert an inhibitory effect on the release of the amyloid Aβ peptide.

2. The method according to claim 1, wherein step (d) comprises the incubation of mammalian cells stably transfected with APP695 with the ligand obtained from steps (a) to (c) and the determination of the production of the amyloid Aβ peptide with polyclonal or monoclonal antibodies.

3. The method according to claim 1, wherein step (d) comprises the administration of the ligand obtained from steps (a) to (c) to transgenic mammals which express the human amyloid Aβ peptide, the collection of a sample from the CNS or the blood and the determination of the production of the amyloid Aβ peptide with polyclonal or monoclonal antibodies.

4. The use of Zn(II) for the preparation of a medicament for the prevention or treatment of Alzheimer's disease.

## Revendications

1. Un procédé pour identifier un composé comme étant un agoniste du cuivre qui se lie au site de liaison du cuivre de APP et exerce un effet inhibiteur sur la libération du peptide Aβ-amyloïde, ce procédé étant **caractérisé par** les étapes suivantes :
a) le contact de APP, ou d'un de ses fragments portant le site de liaison du cuivre, avec une bibliothèque de substances immobilisées ou dissoutes ou des substances naturelles de bas poids moléculaire provenant de microorganismes ou de plantes,
b) quand on utilise une bibliothèque de substances dissoutes ou des substances naturelles de bas poids moléculaire liquides, l'immuno-précipitation compétitif ou "non compétitif" site du complexe de liaison du cuivre /ligand de la solution avec des anticorps spécifiques de APP, ou d'un de ses fragments, ou, lorsque est employée une bibliothèque de substances immobilisées, la libération du ligand par l'addition de sels de cuivre au complexe site de liaison du cuivre /ligand,
c) l'identification du ligand, et
d) la sélection des ligands qui après liaison du site de liaison du cuivre de APP exerce un effet inhibiteur sur la libération du peptide Aβ-amyloïde

2. Le procédé selon la revendication 1, dans lequel l'étape d) comprend l'incubation des cellules mammaires transfectées de façon stable par APP695, à l'aide du ligand obtenu dans les étapes (a) à (c) et la détermination de la production du peptide Aβ-amyloïde à l'aide d'anticorps polyclonaux ou monoclonaux.

3. Le procédé selon la revendication 1, dans lequel l'étape (d) comprend l'administration du ligand obtenu dans les étapes (a) à (c) à des mammifères transgéniques qui expriment le peptide Aβ-amyloïde humain, la collecte d'un échantillon de CNS ou de sang et la détermination de la production de peptide Aβ-amyloïde à l'aide d'anticorps polyclonaux ou monoclonaux.

4. L'utilisation de Zn(II) pour la préparation d'un médicament pour la prévention ou le traitement de la maladie d'Alzheimer.
